# EUROPÄISCHE PATENTANMELDUNG

(11) **EP 3 586 744 A1**
(43) Veröffentlichungstag der Anmeldung: **01.01.2020**
(21) Anmeldenummer: 18180924.5
(22) Anmeldetag: 29.06.2018
(51) Int. Cl.: A61B 5/11

(54) **VERFAHREN UND SYSTEM ZUR GANGANALYSE**

(71) Anmelder: Senmotion GmbH, 12435 Berlin (DE)
(72) Erfinder: Ertelt, Thomas, 12557 Berlin (DE)
(74) Vertreter: Maiwald Patent- und Rechtsanwaltsgesellschaft mbH

(57) **Zusammenfassung**

Die Erfindung betrifft ein Verfahren zur Ganganalyse. Dabei werden zunächst einen Bodenreaktionskraftverlauf (111) enthaltende Daten von einer Kraftmesseinrichtung (110) erhalten. Dann erfolgt ein Bestimmen (S2) einer ersten Näherung des Bodenreaktionskraftverlaufs (111) durch wenigstens eine erste Näherungsfunktion (140), die durch einen Satz von Koeffizienten (141, 142, 143) definiert ist. Dann erfolgt ein Bestimmen (S3) einer zweiten Näherung des Bodenreaktionskraftverlaufs (111) durch eine zweite Näherungsfunktion (150), die auf Basis des Satzes von Koeffizienten (141, 142, 143) der wenigstens einen ersten Näherungsfunktion (140) gebildet wird. Ferner betrifft die Erfindung ein System (100) zur Ganganalyse.

## Beschreibung

### Technisches Gebiet

Die Erfindung betrifft ein Verfahren und ein System zur Ganganalyse. Diese kann insbesondere auf einer kinetischen Betrachtung beruhen.

### Technischer Hintergrund

Eine Ganganalyse untersucht die natürliche Fortbewegungsart eines Menschen, nämlich das Gehen und Laufen. Bei einer kinetischen Betrachtung erfolgt dies üblicherweise über eine Messung der Bodenreaktionskraft, die eine Reaktionskraft des Bodens auf die Kraft ist, die der Körper durch die Füße beim Auftreten auf den Boden überträgt.

Die qualitative und/oder quantitative Beschreibung einer Bodenreaktionskraft findet Anwendung in beispielsweise der Medizin, Biomechanik, Robotik usw. Insbesondere in der klinischen Anwendung kann dies herangezogen werden, um Belastungen, Risiken und pathologische Auffälligkeiten bzw. Veränderungen, insbesondere infolge von Krankheiten oder Verletzungen (akut und chronisch), aufzuzeigen. Im sportmedizinischen, wie auch klinischen Bereich dient der Gang zudem als Indikator für mögliche Belastungsrisiken sowie zur Einschätzung und Abklärung vorhandener Beeinträchtigungen von Patienten bzw. als Maß für den Erfolg einer Therapie.

Eine elementare Kenngröße im diagnostischen Bereich stellt dabei der zeitliche Verlauf der Bodenreaktionskraft dar, welcher in der Regel visuell klassifiziert wird und damit hochgradig von der Erfahrung und dem Wissen eines Diagnostikers abhängig ist. Aufgrund der Komplexität des Bodenreaktionskraftverlaufs und zahlreicher Einflussparameter wird neben der Erfahrung des Diagnostikers insbesondere auf einzelne Kennparameter der Bodenreaktionskraft zurückgegriffen. Hierzu zählen u.a. bei der vertikalen Bodenreaktionskraft eine Maximalkraft, ein Zeitpunkt der Maximalkraft, eine Bodenkontaktzeit und bei der horizontalen Bodenreaktionskraft eine maximale Beschleunigung, maximale Verzögerung, eine Netto Beschleunigung usw. Aus diesen Parametern lassen sich weitere Kenngrößen ermitteln wie z.B. Impuls/Kraftstoß, Symmetrien usw. Trotz dieser Kennparameter und Kenngrößen ist es jedoch noch nicht möglich, die Beschreibung des Bodenreaktionskraftverlaufs zu objektivieren, so dass Fehleinschätzungen auftreten können.

Ein Ansatz zur besseren Objektivierung findet sich beispielsweise in Alaqtash, M., Sarkodie-Gyan, T., Yu, H., Fuentes, O., Brower, R. & Abdelgawad, A. (2011). Automatic classification of pathological gait patterns using ground reaction forces and machine learning algorithms. Dabei werden Bodenreaktionskräfte von Schlaganfallpatienten auf Basis der Kennparameter bzw. Kenngrößen durch einen Maschinelles-Lernen-Algorithmus klassifiziert. Nachteilig daran ist, dass nicht der gesamte Bodenreaktionskraftverlauf betrachtet und analysiert wird, so dass das darin für Schlaganfallpatienten vorgeschlagene Verfahren nicht auf andere Felder übertragbar ist.

### Zusammenfassung der Erfindung

Die Aufgabe der Erfindung ist es daher, eine verbesserte Möglichkeit zur Ganganalyse mit möglichst hoher Objektivität zur Verfügung zu stellen.

Die Aufgabe wird durch den Gegenstand der unabhängigen Ansprüche gelöst. Vorteilhafte Weiterbildungen der Erfindung ergeben sich aus den abhängigen Ansprüchen, der Beschreibung und den begleitenden Figuren.

Ein erster Aspekt der Erfindung betrifft ein Verfahren zur Ganganalyse. Das Verfahren kann beispielsweise mittels einer Datenverarbeitungseinrichtung durchgeführt werden, die einen Prozessor, einen Speicher und ggf. eine Schnittstelle für eine Messeinrichtung usw. aufweisen kann. Das Verfahren kann als Programmanweisungen in dem Speicher gespeichert sein, die von dem Prozessor ausgeführt werden, und die folgenden Schritte umfassen:
- Zunächst werden von einer Kraftmesseinrichtung Daten erhalten, die einen Bodenreaktionskraftverlauf, also eine Bodenreaktionskraft über einen Zeitabschnitt, enthalten. Die Kraftmesseinrichtung kann mit der Datenverarbeitung verbunden sein und so direkt die Daten liefern. Alternativ dazu, können die Daten auch über eine Daten- oder Netzwerkverbindung übertragen werden oder auf einem Datenträger zwischengespeichert werden. Die Daten können zur besseren Vergleichbarkeit auf das Körpergewicht einer Person, deren Gang analysiert werden soll, normiert werden. Dementsprechend kann die Kraft des Bodenreaktionskraftverlaufs auch als prozentualer Anteil des Körpergewichts angegeben sein. Bei Analysen zum Gang, also z.B. Gehen und Laufen, kann der zu beschreibende Kraftverlauf auf eine Kontaktzeit zwischen einem Fuß und dem Boden bzw. der Kraftmesseinrichtung beschränkt sein. Bei einer Analyse mit einem anderen Fokus, wie z.B. bei einer Aufgabe zur Balance, bei Sprüngen, Stopp- oder Seitschrittmanövern, kann der betrachtete Bereich auch auf ein Zeitfenster beschränkt werden.
- Dann erfolgt ein Bestimmen einer ersten Näherung des Bodenreaktionskraftverlaufs durch wenigstens eine erste Näherungsfunktion, die durch einen Satz von Koeffizienten definiert ist.
- Auf Basis des Satzes von Koeffizienten der wenigstens einen ersten Näherungsfunktion wird dann eine zweite Näherung des Bodenreaktionskraftverlaufs durch eine zweite Näherungsfunktion bestimmt, wobei diese durch eine Kombination der Koeffizienten gebildet wird.

In anderen Worten wird ein gewählter Kraftausschnitt, hier der Bodenreaktionskraftverlauf, angenähert, um diesen gemessenen Verlauf in einer möglichst großen Übereinstimmung möglichst objektiv zu beschreiben.

Mit dieser Konfiguration bietet die Erfindung gleich mehrere Vorteile. So wird eine Möglichkeit geschaffen, die Ganganalyse zu objektivieren und insbesondere eine quantifizierbare Beschreibung unabhängig von einem Diagnostiker zu erreichen. Aus der erfindungsgemäßen Ganganalyse lassen sich einheitliche Interventionen und Strategien ableiten, beispielsweise auch für mögliche chirurgische Eingriffe sowie therapeutische und rehabilitive Maßnahmen.

Eine Weiterbildung der Erfindung sieht vor, dass der wenigstens eine Satz von Koeffizienten in einem Koeffizientenpool zusammengefasst werden kann, aus dem einzelne Koeffizienten zum Bilden der zweiten Näherungsfunktion miteinander kombiniert werden können. Je mehr erste Näherungsfunktionen bestimmt werden, desto mehr Sätze von Koeffizienten werden in dem Koeffizientenpool zusammengefasst und stehen für die zweite Näherung zur Verfügung. Durch eine Auswahlmöglichkeit einzelner Koeffizienten aus dem Koeffizientenpool kann der Bodenreaktionskraftverlauf exakt beschrieben werden.

Gemäß einer Weiterbildung kann eine Mehrzahl von zueinander unterschiedlichen ersten Näherungsfunktionen bestimmt werden, die durch den jeweiligen Satz von Koeffizienten definiert sind. Die ersten Näherungsfunktionen können sich z.B. auch nur in einem von mehreren Koeffizienten unterscheiden. Sämtliche Koeffizienten können in dem oben beschriebenen Koeffizientenpool zusammengefasst werden. Durch die Auswahlmöglichkeit aus mehreren Sätzen von Koeffizienten kann die zweite Näherung mit hoher Genauigkeit erfolgen.

In einer anderen Weiterbildung kann vorgesehen sein, dass iterativ wenigstens zwei erste Näherungsfunktionen gewählt werden, aus denen in einem jeweiligen Iterationsschritt eine Summenfunktion und ihre Abweichung vom Bodenreaktionskraftverlauf bestimmt werden, bis die Abweichung ein vorbestimmtes Abbruchkriterium erreicht und daraus die entsprechende erste Näherungsfunktion für die erste Näherung bestimmt wird. Beispielsweise können aus der Summenfunktion der gewählten ersten Näherungsfunktionen Residuen, d.h. die Summe der Abweichungen im Quadrat, bestimmt werden. Durch das Quadrat können kleine Abweichungen in ihrer Wirkung abgeschwächt und große Abweichungen, beispielsweise Abweichungen >1, verstärkt werden. Diese Auswahl bzw. Anpassung kann so oft wiederholt werden, bis ein Minimum der Residuen gegeben ist und die Güte der Funktion ein R² von beispielsweise mindestens 0.8 erreicht. Das Minimum der Residuen, der Werte des R² oder eine Kombination aus beidem können das Abbruchkriterium sein. Die ersten Näherungsfunktionen, die diesem Abbruchkriterium entsprechen, können dann für das Bestimmen der zweiten Näherung durch die zweite Näherungsfunktion herangezogen werden. Dadurch wird mit vergleichsweise geringem Rechenaufwand eine gute erste Näherung als guter Ausgangspunkt für die zweite Näherung erreicht.

Gemäß einer Weiterbildung können die die zweite Näherungsfunktion bildenden Koeffizienten vorab mit wenigstens einem vorbestimmten Datensatz, der eine Mehrzahl von klassifizierten Bodenreaktionskraftverläufen enthalten kann, verglichen werden und daraus validiert werden, ob die Koeffizienten die zweite Näherung ermöglichen. In anderen Worten können die gewählten ersten Näherungsfunktionen bzw. Koeffizienten in einer Art Trainingsset validiert werden. Dazu werden zunächst existierende Bodenreaktionskraftverläufe auf Basis einer z.B. visuellen Analyse voneinander abgegrenzt.

Dabei kann bekannt sein, z.B. durch Beobachtung und einer Zuordnung zu einem Laufstil oder ähnlichem, durch Erkenntnisse aus der Literatur usw., welches Muster eines Bodenreaktionskraftverlaufs einem bestimmten Laufstil entspricht. Es kann dann anhand des Trainingssets geprüft werden, ob es statistisch möglich ist, eine vorbestimmte Klasse, z.B. einen bestimmten Laufstil, tatsächlich zu unterscheiden. So kann z.B. visuell klassifizierten Mustern eine bekannte Kategorie zugeordnet werden, deren Differenzierung in einem Trainingsset geprüft wird.

Eine andere Weiterbildung sieht vor, dass aus den validierten Koeffizienten die zweite Näherungsfunktion iterativ gebildet und die zweite Näherung einer Klasse zugeordnet wird. In anderen Worten, können über den Datensatz Informationen über eine Koeffizientenausprägung in Abhängigkeit zu einer Klassifizierungsgruppe, wie z.B. Laufstil, Sportart, Pathologie ähnliches, erhalten werden. Diese validierten Koeffizienten können nun verwendet werden, um eine möglichst gute Übereinstimmung mit dem zu analysierenden Bodenreaktionskraftverlauf zu erhalten. D.h., dass ein registrierter Bodenreaktionskraftverlauf auf Basis der vorhandenen Koeffizienten approximiert werden kann. Ein Abbruchkriterium kann eine Minimierung des Residuums sein. Es ist nun auch bekannt, welche grundsätzliche Ausprägung die Koeffizienten in einer jeweiligen Klasse aufweisen, so dass für den Bodenreaktionskraftverlauf mit einer entsprechenden Wahrscheinlichkeit die Klasse bestimmt werden kann.

Gemäß einer Weiterbildung kann die erste Näherungsfunktion eine Normalverteilung oder Gauß-Funktion sein. Diese lassen sich einfach bestimmen und weisen eine geringe Komplexität auf. Dennoch kann damit eine gute erste Näherung erreicht werden.

Eine andere Weiterbildung sieht vor, dass die zweite Näherungsfunktion eine Summenfunktion der wenigstens einen ersten Näherungsfunktion sein kann. Diese lässt sich einfach bilden und erlaubt, z.B. über ein Residuum als Abbruchkriterium, eine iterative Näherung mit geringem Rechenaufwand.

In einer Weiterbildung kann der Satz von Koeffizienten einen Maximalwert, einen Mittelwert und/oder eine Breite der ersten Näherungsfunktion umfassen. In anderen Worten kann jede erste Näherungsfunktion drei Koeffizienten umfassen. Dadurch lässt sich beispielsweise eine Normalverteilung oder Gauß-Funktion vollständig definieren.

Gemäß einer anderen Weiterbildung kann eine Anzahl der bestimmten ersten Näherungsfunktion zwischen 1 und 20, bevorzugt zwischen 1 und 15, besonders bevorzugt auf genau 8, festgelegt werden. Die ersten Näherungsfunktionen können beispielsweise durch drei Koeffizienten definiert sein, so dass ich für zwei erste Näherungsfunktionen 2·3=6, für drei erste Näherungsfunktionen 3·3=9 usw. Koeffizienten ergeben. Es hat sich gezeigt, dass für eine klinische Anwendung der Ganganalyse besonders gute Ergebnisse zur Beschreibung des Bodenreaktionskraftverlaufs mit acht ersten Näherungsfunktionen, also 8·3=24 Koeffizienten erreicht werden können. Jedoch können in Abhängigkeit der Komplexität auch 15 oder mehr erste Näherungsfunktionen bestimmt werden.

Ein zweiter Aspekt der Erfindung betrifft ein Ganganalysesystem. Dieses kann mittels einer Datenverarbeitungseinrichtung umgesetzt sein und ist vorzugsweise dazu eingerichtet, das vorstehend beschriebene Verfahren in einer oder mehreren Ausführungsvarianten durchzuführen. Das Ganganalysesystem weist auf:
- Eine Kraftmesseinrichtung, die dazu eingerichtet ist, einen Bodenreaktionskraftverlauf zu erfassen. Diese kann z.B. eine Kraftmessplatte, ein instrumentiertes Laufband, instrumentierte Einlegesohlen oder ähnliches aufweisen.
- Eine elektronische Auswerteeinrichtung, die z.B. einen Prozessor und einen Speicher umfassen kann, und die dazu eingerichtet ist,
- den erfassten Bodenreaktionskraftverlauf durch wenigstens eine erste Näherungsfunktion anzunähern, die durch einen Satz von Koeffizienten definiert ist, und
- den Bodenreaktionskraftverlauf durch eine zweite Näherungsfunktion weiter anzunähern, die auf Basis des Satzes von Koeffizienten gebildet ist.

So wird eine Möglichkeit geschaffen, die Ganganalyse zu objektivieren und insbesondere eine quantifizierbare Beschreibung unabhängig von einem Diagnostiker zu erreichen.

Eine Weiterbildung der Erfindung sieht vor, dass das Ganganalysesystem ferner eine Datenbankeinrichtung aufweisen kann, die dazu eingerichtet sein kann, eine Kombination von die zweite Näherungsfunktion bildenden Koeffizienten einem vorbestimmten Gangverhalten und/oder einer Gangauffälligkeit zuzuordnen. Beispielsweise kann die Datenbankeinrichtung den oben beschriebenen vorbestimmten Datensatz umfassen und dazu eingerichtet sein, dass die Koeffizienten anhand des Datensatzes, insbesondere durch die Auswerteeinrichtung, zu validieren und/oder zu klassifizieren.

Gemäß einer anderen Weiterbildung kann das Ganganalysesystem ferner wenigstens ein neuronales Netz aufweisen, das dazu eingerichtet ist, die Koeffizienten als Eingangsgröße zu erhalten und eine Klasseninformation als Ausgangsgröße zu erzeugen. Das neuronale Netz kann mehrschichtig und/oder faltend sein, wobei die Koeffizienten einer Eingangsschicht zugeführt und die Klasseninformation, z.B. Laufstil, Sportart, Pathologie oder ähnliches, von der Ausgangsschicht ausgegeben werden. Das neuronale Netz kann auch bei sehr komplexen Bodenreaktionskraftverläufen sogar graduelle Unterschiede zwischen den einzelnen Koeffizienten und Mustern erkennen und kann deshalb gegenüber statistischen Verfahren, wie Diskriminanz- oder Clusteranalysen, vorteilhaft sein.

Ein dritter Aspekt der Erfindung betrifft ein Programmelement. Dieses veranlasst, wenn es mittels eines Prozessors einer elektronischen Auswerteeinrichtung ausgeführt wird, die Auswerteeinrichtung dazu, beispielsweise das vorstehend beschriebene Verfahren in einer oder mehreren Ausführungsvarianten durchzuführen. Insbesondere veranlasst es die Durchführung der folgenden Schritte:
- Erhalten von einen Bodenreaktionskraftverlauf enthaltenden Daten von einer Kraftmesseinrichtung,
- Bestimmen einer ersten Näherung des Bodenreaktionskraftverlaufs durch wenigstens eine erste Näherungsfunktion, die durch einen Satz von Koeffizienten definiert ist, und
- Bestimmen einer zweiten Näherung des Bodenreaktionskraftverlaufs durch eine zweite Näherungsfunktion, die auf Basis des Satzes von Koeffizienten der ersten Näherungsfunktion gebildet wird.

Die sich daraus ergebenden Vorteile sind vorstehend für das Verfahren sowie das Ganganalysesystem beschrieben.

Ein vierter Aspekt der Erfindung betrifft ein computerlesbares Medium, auf dem das vorstehend beschriebene Programmelement gespeichert ist.

### Kurze Beschreibung der Figuren

Im Folgenden werden bevorzugte Ausführungsformen der Erfindung unter Bezugnahme auf die begleitenden Figuren erläutert. Es zeigen:
- Figur 1: ein erfindungsgemäßes System zur Ganganalyse, das sich zur Durchführung eines erfindungsgemäßen Verfahrens zur Ganganalyse eignet,
- Figur 2: einen beispielhaften Bodenreaktionskraftverlauf mit seiner ersten und zweiten Näherung,
- Figur 3: eine beispielhafte erste Näherungsfunktion, die durch drei Koeffizienten definiert ist, und
- Figur 4: ein Flussdiagramm eines erfindungsgemäßen Verfahrens zur Ganganalyse.

Die Figuren sind lediglich schematische Darstellungen und dienen nur der Erläuterung der Erfindung. Gleiche oder gleichwirkende Elemente sind durchgängig mit den gleichen Bezugszeichen versehen.

### Bevorzugte Ausführungsformen der Erfindung

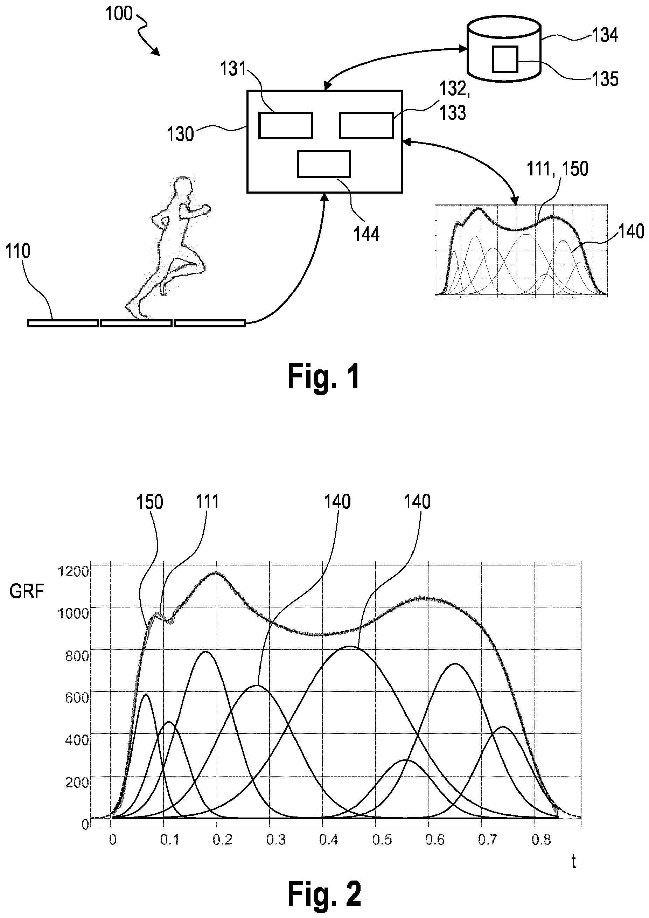

Figur 1 zeigt einen schematischen Aufbau eines Ganganalysesystems 100, das sich insbesondere dazu eignet, Belastungen, Risiken und pathologische Auffälligkeiten bzw. Veränderungen, insbesondere infolge von Krankheiten oder Verletzungen, aufzuzeigen.

Das Ganganalysesystem 100 weist zunächst eine Kraftmesseinrichtung 110 zur messtechnischen Erfassung einer Bodenreaktionskraft über einen Zeitabschnitt, also eines Bodenreaktionskraftverlaufs 111, auf. Die Kraftmesseinrichtung 110 ist hier exemplarisch als Kraftmessplatte ausgeführt und liefert elektronische Daten, die den Bodenreaktionskraftverlauf 111 enthalten. Des Weiteren verfügt das Ganganalysesystem 100 über eine elektronische Auswerteeinrichtung 130, die einen Prozessor 131 und einen Speicher 132 aufweist und zum Erhalten der Daten der Kraftmesseinrichtung 110, z.B. über eine Datenschnittstelle, eingerichtet ist. In dem Speicher 132 sind Programmanweisungen bzw. ein Programmelement 133 gespeichert, die von dem Prozessor 131 ausgeführt werden können und in denen wenigstens ein Künstliches-Intelligenz-Modul und/oder ein neuronales Netz mit einer Eingangsschicht, einer oder mehreren Zwischenschichten und einer Ausgangsschicht implementiert ist. Die Auswerteeinrichtung 130 weist zudem eine Datenbankeinrichtung 134 auf oder ist mit dieser verbunden. In der Datenbankeinrichtung 134 ist wenigstens ein vorbestimmter Datensatz 135 mit Vergleichsdaten zur Auswertung bzw. Klassifizierung des Bodenreaktionskraftverlaufs 111 verfügbar.

Anhand von Figur 2, die in einem Diagramm einen beispielhaften, von der Kraftmesseinrichtung 110 als Daten gelieferten Bodenreaktionskraftverlauf 111 zeigt, wird im Folgenden ein beispielhafter Betrieb des Ganganalysesystems 100 erläutert. In dem Diagramm stellt die vertikale Achse die Bodenreaktionskraft (engl. ground reaction force) dar, die deshalb mit GRF gekennzeichnet ist. Die horizontale Achse des Diagramms stellt die Zeit dar, die deshalb mit t gekennzeichnet ist.

Zunächst werden die von der Kraftmesseinrichtung 110 erhaltenen Daten auf das Körpergewicht derjenigen Person normiert, deren Gang analysiert werden soll. Dies kann anhand von Gewichtsdaten beispielsweise automatisch oder durch eine Eingabe über eine Benutzerschnittstelle oder ähnliches erfolgen.

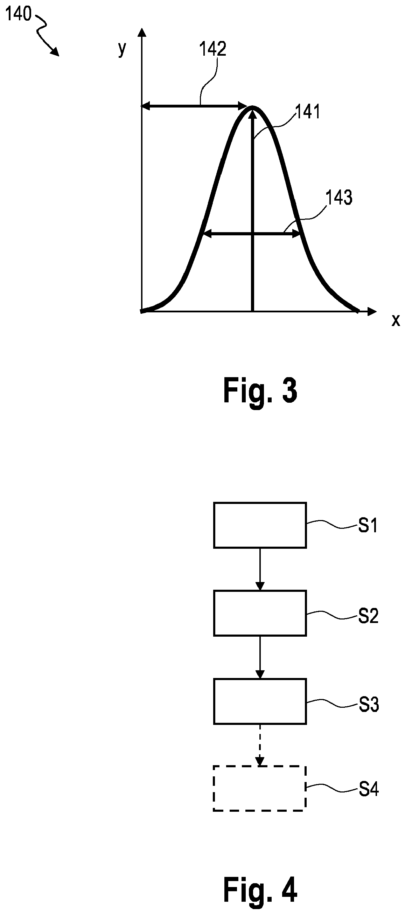

Dann wird mittels der Auswerteeinrichtung 130 eine erste Näherung des Bodenreaktionskraftverlauf 111 bestimmt, wozu mehrere erste Näherungsfunktionen 140 gewählt werden. In dem in Figur 2 gezeigten Ausführungsbeispiel sind für die dortige klinische Ganganalyse genau acht erste Näherungsfunktionen 140 gewählt worden. Die ersten Näherungsfunktionen 140 stellen Normalverteilungen bzw. Gauß-Funktionen dar, unterscheiden sich voneinander und sind durch einen Satz von Koeffizienten 141, 142, 143 definiert, die einen Maximalwert, eine Position, z.B. relativ zu einer X-Achse, insbesondere z.B. die Position eines Maximalwerts und/oder einen Mittelwert, und eine Breite der jeweiligen ersten Näherungsfunktion 140 darstellen (vgl. Figur 3). Die ersten Näherungsfunktionen 140 werden in einem iterativen Verfahren gewählt, indem in jedem Iterationsschritt eine Summenfunktion und ihre Abweichung von dem Bodenreaktionskraftverlauf 111 bestimmt werden, bis die Abweichung ein vorbestimmtes Abbruchkriterium erreicht und daraus die entsprechende erste Näherungsfunktion für die erste Näherung bestimmt wird. In diesem Ausführungsbeispiel werden aus der Summenfunktion der ersten Näherungsfunktionen 140 Residuen, d.h. die Summe der Abweichungen im Quadrat, bestimmt. Sind diese Abweichungen einer ersten Näherungsfunktion 140 zu groß, wird eine andere gewählt. Dies wird so oft wiederholt, bis ein Minimum der Residuen gegeben ist und die Güte der Funktion ein R² von beispielsweise mindestens 0.8 erreicht. Die Kombination aus Minimum der Residuen und die Werte des R² sind das Abbruchkriterium für das iterative Verfahren. Sind die ersten Näherungsfunktionen 140 für die erste Näherung somit gefunden, werden die Koeffizienten 141, 142, 143 aller ersten Näherungsfunktionen 140 in einem Koeffizientenpool 144 (siehe Figur 1) zusammengefasst, wobei in diesem Ausführungsbeispiel dadurch 8·3 = 24 Koeffizienten in dem Koeffizientenpool 144 enthalten sind.

Auf Basis der Koeffizienten 141, 142, 143 der ersten Näherungsfunktionen 140, die hier in dem Koeffizientenpool 144 enthalten sind, wird eine zweite Näherung des Bodenreaktionskraftverlaufs 111 durch eine zweite Näherungsfunktion 150 bestimmt. Dies erfolgt durch das Zuführen der Koeffizienten 141, 142, 143 zu der Eingangsschicht des neuronalen Netzes der Auswerteeinrichtung 130. Die zweite Näherungsfunktion 150 ist eine Summenfunktion der ersten Näherungsfunktionen und ist aus einem Teil oder aus allen Koeffizienten 141, 142, 143 des Koeffizientenpools 144 gebildet. In Figur 2 ist die zweite Näherungsfunktion 150 als gestrichelte Linie eingezeichnet, wobei ein Vergleich mit dem Bodenreaktionskraftverlauf 111 zeigt, dass die zweite Näherung diesen äußerst präzise beschreibt, da diese annähernd deckungsgleich sind. Die Koeffizienten 141, 142, 143 des Koeffizientenpools 144, die zur Bildung der zweiten Näherungsfunktion 150 herangezogen werden vorab mit dem Datensatz 135 verglichen, der eine Mehrzahl von bereits klassifizierten Vergleichs-Bodenreaktionskraftverläufen enthält. Anhand dieses Vergleichs wird ermittelt, ob es statistisch möglich ist, eine vorbestimmte Klasse, z.B. einen bestimmten Laufstil, tatsächlich zu unterscheiden. Damit werden die Koeffizienten 141, 142, 143 des Koeffizientenpools 144 vorab validiert. Dann wird aus den anhand des Datensatzes 135 validierten Koeffizienten 141, 142, 143 die zweite Näherungsfunktion 150 in einem iterativen Verfahren gebildet und die zweite Näherung einer Klasse zugeordnet. Dabei werden die Koeffizienten 141, 142, 143 so oft kombiniert, bis ein Abbruchkriterium erreicht wird, das in einer Minimierung eines Residuums bestehen kann. Daraus kann der angenäherte Bodenreaktionskraftverlauf 111 eine Ausprägung der Koeffizienten 141, 142, 143 aufweisen, die einer jeweiligen Klasse zugeordnet werden können. Als Ergebnis wird von der Ausgabeschicht des neuronalen Netzes eine Beschreibung und Klassifikation des Bodenreaktionskraftverlaufs 111 ausgegeben. Dieses Ergebnis lässt eine objektive und valide Ganganalyse zu.

Figur 3 zeigt zur besseren Veranschaulichung eine exemplarische erste Näherungsfunktion, die durch die drei Koeffizienten, nämlich dem Maximalwert 141, der Position, 142 und der Breite 143 definiert ist.

Anhand von Figur 4, die ein Flussdiagramm zeigt, wird nun ein erfindungsgemäßes Verfahren zur Ganganalyse erläutert. Zunächst werden in einem Schritt S1 die Daten, die den Bodenreaktionskraftverlauf 111 enthalten, von der Kraftmesseinrichtung 110 erhalten. Dann wird in einem Schritt S2 eine erste Näherung des Bodenreaktionskraftverlaufs 111 durch eine oder mehrere der ersten Näherungsfunktionen 140 bestimmt, die durch die Koeffizienten 141, 142, 143 definiert sind. In einem Schritt S3 wird eine zweiten Näherung des Bodenreaktionskraftverlaufs 111 durch die zweite Näherungsfunktion 150 bestimmt, die auf Basis der Koeffizienten 141, 142, 143 der einen oder der mehreren ersten Näherungsfunktionen 140 gebildet wird. In einem optionalen Schritt S4 werden die Koeffizienten 141, 142, 143 mit wenigstens einem vorbestimmten Datensatz 135, der eine Mehrzahl von klassifizierten Bodenreaktionskraftverläufen enthält, verglichen. Anhand dieses Vergleichs wird validiert, ob sich die Koeffizienten 141, 142, 143 die zweite Näherung ermöglichen. Weiter optional, werden die Koeffizienten 141, 142, 143 durch den Vergleich mit dem Datensatz 135 einem vorbestimmten Gangverhalten, einer Gangauffälligkeit, und/oder einer anderen Fuß-Boden-Interaktion zugeordnet.

Obwohl die vorliegende Erfindung anhand bevorzugter Ausführungsbeispiele vorstehend beschrieben wurde, ist sie nicht darauf beschränkt, sondern auf vielfältige Art und Weise modifizierbar. Insbesondere lässt sich die vorliegende Erfindung in mannigfaltiger Weise verändern oder modifizieren, ohne vom Kern der Erfindung abzuweichen.

Ergänzend sei darauf hingewiesen, dass "umfassend" und "aufweisend" keine anderen Elemente oder Schritte ausschließt und "eine" oder "ein" keine Vielzahl ausschließt.

Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkungen anzusehen.

### Bezugszeichenliste

- 100: System zur Ganganalyse
- 110: Kraftmesseinrichtung
- 111: Bodenreaktionskraftverlauf
- 130: Auswerteeinrichtung
- 131: Prozessor
- 132: Speicher
- 133: Programmelement
- 134: Datenbankeinrichtung
- 135: vorbestimmter Datensatz
- 140: erste Näherungsfunktion
- 141: erster Koeffizient (z.B. Maximalwert)
- 142: zweiter Koeffizient (z.B. Position)
- 143: dritter Koeffizient (z.B. Breite)
- 144: Koeffizientenpool
- 150: zweite Näherungsfunktion

## Patentansprüche

1. Verfahren zur Ganganalyse, mit den Schritten:
- Erhalten (S1) von einen Bodenreaktionskraftverlauf (111) enthaltenden Daten von einer Kraftmesseinrichtung (110),
- Bestimmen (S2) einer ersten Näherung des Bodenreaktionskraftverlaufs (111) durch wenigstens eine erste Näherungsfunktion (140), die durch einen Satz von Koeffizienten (141, 142, 143) definiert ist, und
- Bestimmen (S3) einer zweiten Näherung des Bodenreaktionskraftverlaufs (111) durch eine zweite Näherungsfunktion (150), die auf Basis des Satzes von Koeffizienten (141, 142, 143) der wenigstens einen ersten Näherungsfunktion (140) gebildet wird.

2. Verfahren nach Anspruch 1, wobei der wenigstens eine Satz von Koeffizienten (141, 142, 143) in einem Koeffizientenpool (144) zusammengefasst wird, aus dem einzelne Koeffizienten (141, 142, 143) zum Bilden der zweiten Näherungsfunktion (150) miteinander kombiniert werden können.

3. Verfahren nach Anspruch 1 oder 2, wobei eine Mehrzahl von zueinander unterschiedlichen ersten Näherungsfunktionen (140) bestimmt wird, die durch den jeweiligen Satz von Koeffizienten (141, 142, 143) definiert sind.

4. Verfahren nach einem der vorhergehenden Ansprüche, wobei iterativ wenigstens zwei erste Näherungsfunktionen (140) gewählt werden, aus denen in einem jeweiligen Iterationsschritt eine Summenfunktion und ihre Abweichung vom Bodenreaktionskraftverlauf (111) bestimmt werden, bis die Abweichung ein vorbestimmtes Abbruchkriterium erreicht und daraus die entsprechende erste Näherungsfunktion (140) für die erste Näherung bestimmt wird.

5. Verfahren nach einem der vorhergehenden Ansprüche, wobei die die zweite Näherungsfunktion (150) bildenden Koeffizienten (141, 142, 143) vorab mit wenigstens einem vorbestimmten Datensatz (135), der eine Mehrzahl von klassifizierten Bodenreaktionskraftverläufen enthält, verglichen (S4) werden und daraus validiert wird, ob die Koeffizienten (141, 142, 143) die zweite Näherung ermöglichen.

6. Verfahren nach Anspruch 5, wobei aus den validierten Koeffizienten (141, 142, 143) die zweite Näherungsfunktion (150) iterativ gebildet und die zweite Näherung einer Klasse zugeordnet wird.

7. Verfahren nach einem der vorhergehenden Ansprüche, wobei die erste Näherungsfunktion (140) eine Normalverteilung oder Gauß-Funktion ist.

8. Verfahren nach einem der vorhergehenden Ansprüche, wobei die zweite Näherungsfunktion (150) eine Summenfunktion der wenigstens einen ersten Näherungsfunktion (140) ist.

9. Verfahren nach einem der vorhergehenden Ansprüche, wobei der Satz von Koeffizienten einen Maximalwert (141), eine Position (142) und/oder eine Breite (143) der ersten Näherungsfunktion (140) umfasst.

10. Verfahren nach einem der vorhergehenden Ansprüche, wobei eine Anzahl der bestimmten ersten Näherungsfunktion (140) zwischen 1 und 20, bevorzugt zwischen 1 und 15, besonders bevorzugt auf genau 8, festgelegt wird.

11. Ganganalysesystem (100), aufweisend
- eine Kraftmesseinrichtung (110), die dazu eingerichtet ist, einen Bodenreaktionskraftverlauf (111) zu erfassen,
- eine elektronische Auswerteeinrichtung (130), die dazu eingerichtet ist,
- den erfassten Bodenreaktionskraftverlauf (111) durch wenigstens eine erste Näherungsfunktion (140) anzunähern, die durch einen Satz von Koeffizienten (141, 142, 143) definiert ist, und
- den Bodenreaktionskraftverlauf (111) durch eine zweite Näherungsfunktion (150) weiter anzunähern, die auf Basis des Satzes von Koeffizienten (141, 142, 143) gebildet ist.

12. Ganganalysesystem (100) nach Anspruch 11, ferner aufweisend eine Datenbankeinrichtung (134), die dazu eingerichtet ist, eine Kombination von die zweite Näherungsfunktion (150) bildenden Koeffizienten (141, 142, 143) einem vorbestimmten Gangverhalten, einer Gangauffälligkeit, und/oder einer anderen Fuß-Boden-Interaktion zuzuordnen.

13. Ganganalysesystem (100) nach einem der Ansprüche 11 oder 12, ferner aufweisend wenigstens ein neuronales Netz, das dazu eingerichtet ist, die Koeffizienten (141, 142, 143) als Eingangsgröße zu erhalten und eine Klasseninformation als Ausgangsgröße zu erzeugen.

14. Programmelement (132), das, wenn es mittels eines Prozessors (131) einer elektronischen Auswerteeinrichtung (130) ausgeführt wird, die Auswerteeinrichtung (130) dazu veranlasst, die folgenden Schritte durchzuführen:
- Erhalten von einen Bodenreaktionskraftverlauf (111) enthaltenden Daten von einer Kraftmesseinrichtung (110),
- Bestimmen einer ersten Näherung des Bodenreaktionskraftverlaufs (111) durch wenigstens eine erste Näherungsfunktion (140), die durch einen Satz von Koeffizienten (141, 142, 143) definiert ist, und
- Bestimmen einer zweiten Näherung des Bodenreaktionskraftverlaufs (111) durch eine zweite Näherungsfunktion (150), die auf Basis des Satzes von Koeffizienten (141, 142, 143) der ersten Näherungsfunktion (140) gebildet wird.

15. Computerlesbares Medium, auf dem ein Programmelement (132) nach Anspruch 14 gespeichert ist.
